# EUROPEAN PATENT SPECIFICATION

(11) **EP 1 349 553 B1**
(45) Date of publication and mention of the grant of the patent: **06.09.2006**
(21) Application number: 02715601.7
(22) Date of filing: 14.01.2002
(51) Int. Cl.: A61K 31/575, A61K 31/045, A61P 37/02, A61P 29/00, A61P 17/00

(54) **DIHYDRO-TRITERPENES IN THE TREATMENT OF VIRAL INFECTIONS, CARDIOVASCULAR DISEASE, INFLAMMATION, HYPERSENSITIVITY OR PAIN**
DIHYDROTRITERPENE BEI DER BEHANDLUNG VON VIRUSINFEKTIONEN, HERZ UND KREISLAUF KRANKHEITEN, ENTZÜNDUNGEN, ÜBEREMPFINDLICHKEITEN ODER SCHMERZEN
DIHYDRO-TRITERPENES DANS LE TRAITEMENT D'INFECTIONS VIRALES, DE MALADIES CARDIOVASCULAIRES, D'INFLAMMATIONS, D'HYPERSENSIBILITE OU DE DOULEURS

(30) Priority: 12.01.2001 DK 200100049; 16.01.2001 US 261168 P
(43) Date of publication of application: 08.10.2003
(73) Proprietor: Bsp Pharma, 2100 Copenhagen (DK)
(72) Inventor: WEIDNER, Morten, Sloth, DK-2830 Virum (DK)
(74) Representative: Plougmann & Vingtoft A/S
(86) International application number: PCT/IB2002/000081
(87) International publication number: WO 2002/055087

(56) References cited:
- WO-A-01/03712
- WO-A-95/35103
- WO-A-98/32443
- FR-A- 2 770 400
- GB-A- 932 662
- US-A- 4 748 161
- TOSHIHIRO ITOH ET AL: "Sterols,methylsterols, and triterpene alcohols in three THEACEAE and some other vegetable oils." LIPIDS, vol. 9, no. 3, 1974, pages 173-184, XP002902463
- BUDZIKIEWICZ H ET AL : "Mass spectrometry in structural and stereochemical problems. XXXII. Pentacyclic triterpenes." JOURNAL AMERICAN SOC., vol. 85, 1963, pages 3688-3699, XP002902437
- TOSHIHIRO AKIHISA ET AL : "Triterpene alcohols from camellia and sasanqua oils and their anti-inflammatory effects." CHEM. PHARM. BULL. , vol. 45, no. 12, 1997, pages 2016-2023, XP002902438
- YASUKAWA K ET AL: "SOME LUPANE-TYPE TRITERPENES INHIBIT TUMOR PROMOTION BY 12-O-TETRADECANOYLPHORBOL-13-ACETATE IN TWO-STAGE CARCINOGENESIS IN MOUSE SKIN" PHYTOMEDICINE, GUSTAV FISCHER VERLAG, STUTTGART, DE, vol. 4, 1995, pages 309-313, XP000610925 ISSN: 0944-7113
- DATABASE STN INTERNATIONAL [Online] file caplus; DEL CARMEN RECIO M ET AL: "Structural requirements for the anti-inflammatory activity of natural triterpenoids." retrieved from CAPLUS, accession no. 1995:544934 Database accession no. 122:281461 XP002902439 & PLANTA MED., vol. 61, no. 2, 1995, pages 182-185,
- DATABASE WPI Section Ch, Week 199334 Derwent Publications Ltd., London, GB; Class D21, AN 1993-269741 XP002902440 & JP 05 186326 A (SUNSTAR CHEM IND), 27 July 1993 (1993-07-27)
- DE DECKERE E A M ET AL: "Minor constituents of rice bran oil as functional foods." NUTRITION REVIEWS, vol. 54, no. 11, November 1996 (1996-11), pages s120-s126, XP002902441
- TOSHIHIRO AKIHISA ET AL: "Inhibitory effects of triterpenoids and sterols on human immunodeficiency Virus-1 reverse transcriptase." LIPIDS, vol. 36, no. 5, May 2001 (2001-05), pages 507-512, XP002902442

## Description

### FIELD OF THE INVENTION

The present invention relates to compositions comprising dihydrobutyrospermol, dihydrolupeol or dihydroparkeol, or mixtures thereof. The composition may be formulated with pharmaceutically acceptable carriers and/or excipients for oral, parenteral or topical administration. The composition may be used as a pharmaceutical, a dietary supplement or a cosmetic. The invention also relates to the use of such compositions for the preparation of a medicament for immunomodulation such as suppression of hypersensitivity, inflammatory reactions and viral infections.

### BACKGROUND OF THE INVENTION

Hypersensitivity is defined as a state of altered reactivity in which the body reacts with an exaggerated immune response to a substance (antigen). Hypersensitivity may be caused by exogenous or endogenous antigens.

Hypersensitivity reactions underlie a large number of diseases. Among these, allergic and autoimmune conditions are of great importance. A classification of hypersensitivity diseases is given in the textbook Clinical Medicine (Kumar, P. and Clark, M.: "Clinical Medicine", 3rd edition, p. 147-150, 1994, Bailliere Tindall, London). Typically, the hypersentivity reactions are categorised In four types:
- Type I hypersensitivity reactions (IgE mediated allergic reactions) are caused by allergens (specific exogenous antigens), e.g. pollen, house dust, animal dandruff, moulds, etc. Allergic diseases in which type I reactions play a significant role include asthma, eczema (atopic dermatitis), urticaria, allergic rhinitis and anaphylaxis.
- Type II hypersensitivity reactions are caused by cell surface or tissue bound antibodies (IgG and IgM) and play a significant role in the pathogenesis of myasthenia gravis, Goodpasture's syndrome and Addisonian pernicious anaemia.
- Type III hypersensitivity reactions (immune complex) are caused by autoantigens or exogenous antigens, such as certain bacteria, fungi and parasites. Diseases in which type III hypersensitivity reactions play a significant role include lupus erythematosus, rheumatoid arthritis and glomerulonephritis.
- Type IV hypersensitivity reactions (delayed) are caused by cell or tissue bound antigens. This type of hypersensitivity plays a significant role in a number of conditions, e.g. graft-versus-host disease, leprosy, contact dermatitis and reactions due to insect bites.

Type I to type IV hypersensitivity reactions are all classic allergic reactions, which may lead to histamine release. However, hypersensitivity reactions are also those, where histamine release is triggered through the direct action of "triggering substances" with the cellular membrane. Examples of "triggering substances" are, but not limited to, toxins, food constituents and certain drugs.

A number of drug classes are available for the treatment of hypersensitivity reactions. Among these, the corticosteroids are some of the most widely used drugs. Corticosteroids primarily exert their pharmacological action by non-selectively inhibiting the function and proliferation of different classes of immune cells resulting in suppression of hypersensitivity reactions. Unfortunately, the corticosteroids are associated with a number of serious side effects, e.g. immuno-suppression, osteoporosis and skin atrophy.

Viruses are small infectious agents that contain either DNA or RNA. Because viruses are metabolically inert, they must live intracellularly, using a host cell for synthesis of viral proteins and nucleic acid. Viruses have a central nucleic core surrounded by a protein coat that is antigenically unique for a particular virus. Some viruses also possess an envelope consisting of protein and lipid.

The two major classes of viruses are DNA viruses and RNA viruses.

A relatively limited number of drugs are available for the treatment of viral infections:
- Interferons (INF's) are potent cytokines that possess antiviral, immunomodulating and antlproliferative actions. These proteins are synthesized by cells in response to various inducers and in turn cause biochemical changes leading to an antiviral state in cells of the same species. Three major classes of interferons with significant antiviral activity are currently recognised: alpha (of which there are more than 24 individual species), beta and gamma. Clinically used recombinant alpha interferons are non-glycosylated proteins of approximately 19.5 kDa.
   Both recombinant and natural alpha interferons are approved in the United States, depending on the specific interferon type, for the treatment of condyloma acuminatum, chronic hepatitis C, chronic hepatitis B, Kaposi's sarcoma in HIV-infected patients and multiple sclerosis.
- Acyclovir is an acyclic guanine nucleoside analogue, which is used topically and systemically for the treatment of herpes viruses. Valacyclovir Is a prodrug of acyclovir.
- Other anti-herpes virus agents are famciclovir, penciclovir and ganciclovir which are also acyclic guanine nucleoside analogues. Other anti-herpes virus agents are foscarnet which is an inorganic pyrophosphate analogue, idoxuridine which is an iodinated thymidine analogue, trifluridine which is a flourinated pyrimidine nucleoside, vidarabine which is an adenosine analogue, and sorivudine which is a pyrimidine nucleoside analogue.
- Among the currently available antiretroviral agents, zidovudine is a thymidine analogue (active against HIV-1, HIV-2 and HTLV-1), didanosine is a purine nucleoside analogue (active against HIV-1 and HIV-2), stavudine is a thymidine nucleoside analogue (active against HIV-1) and zalcitabine is a cytosine nucleoside analogue (active against HIV-1).
- Among other antiviral agents are amantadine and rimantadine which are uniquely configured tricyclic amines. Both agents are used for the treatment of influenza A virus infections.The triterpenes constitute a large diverse group of natural products derived from squalene (Abe, F. and Yamauchi, T. 1987. *Chem. Pharm. Bull.* **35,** 1833-1838). In excess of 4000 triterpenes have been isolated so far and more than 40 different skeletal types have been isolated. Triterpenes are widespread in the plant kingdom and occur in numerous plants.

The triterpene alcohols butyrospermol, lupeol and parkeol are widely occurring in many plants induding *Theaceae (*e.g. *Camellia japonica* L.*, Camellia sasanqua* Thunb. and *Thea sinensis* L.) and *Butyrospermum parkii* (karite tree).

The triterpene alcohols dihydrobutyrospermol (T. Itoh, T. Tamura and T. Matsumoto, *Lipids*, 1974 vol. 9, No. 3, 173-184), dihydrolupeol (H. Budzikiewlcz, J. M. Wilson and C. Djerassi, *J. Am. Soc.,* 1963, vol. 85, 3688-3699) and/or dihydroparkeol (T. Itoh, T. Tamura and T. Matsumoto, *Lipids*, 1974 vol. 9, No. 3, 173-184) are hydrogenation products of butyrospermol, lupeol and parkeol, respectively.

Numerous pharmacological actions have been attributed to triterpenes. Such actions are highly diverse and depend on the specific structures of the triterpenes.

Pharmacological effects or any pharmaceutical, dietary or cosmetic use of the triterpenes dihydrobutyrospermol, dihydrolupeol and dihydroparkeol, or derivatives thereof have not previously been described in the literature.

Thus, to the inventor's best knowledge, compositions according to the present invention which comprises a mixture of triterpenes comprising two or three of the triterpenes selected from the group consisting of dihydrobutyrospermol, dihydrolupeol and dihydroparkeol, either in the form of the free alcohols in the form of derivatives such as esters and ethers, have never been disclosed before.

WO 01/03712 describes compositions comprising a mixture of butyrospermol, lupeol and parkeol.

Toshihiro et al. Lipids, 9:1974;173-184 discloses purification of several triterpenes from different seed oils.

GB 932 662 discloses the cicatrizing and bacterial properties of compositions comprising butyrospermol isolated from *Butyrospermum parkii*.

Toshihiro et al. Chem. Pharm. Bull. 45:1997:2016-2023 discloses triterpene alcohols obtained from *Camellia* and their anti-inflammatory properties.

WO 95/35103 discloses pharmaceutical compositions comprising β-lupeol derivatives for the treatment of viral infections and inflammation.

Yasukawa et al. in "Some lupane-type trlterpenes inhibit tumor promotion by 12-0-tetradecanoylphorbol-13-acetate in two stage carcinogenesis in mouse skin phytomedicine", Gustav Fisher Verlag, Stuttgart, DE, vol. 4, 1995, pages 309-313.

There is a strong need for new antiviral and anti-inflammatory drugs due to a lack of efficacy of existing therapeutic agents and because of a number of unpleasant side effects related thereto.

### SUMMARY OF THE INVENTION

It has been found that dihydrobutyrospermol (7(8)-en-4,4,14-trimetyl-cholestan-3-ol), dihydrolupeol (lupan-3-ol) and/or dihydroparkeol (9(11)-en-4,4,14-trimetyl-cholestan-3-ol), wherein said triterpenes may be in the form of free alcohols or derivatives thereof, especially cinnamic add esters, acetic add esters or fatty add esters, possess surprising anti-inflammatory effects relevant to anti-inftammatory and hypersensitivity diseases, and surprising antiviral effects relevant to the treatment of viral infections. Compared to the existing antiviral drugs, dihydrobutyrospermol, dihydrolupeol and/or dihydroparkeol have the advantage of being active against a broad range of viruses and not being associated with any serious side effects. Furthermore, dihydrobutyrospermol, dihydrolupeol and/or dihydroparkeol inhibit the secretion of inflammatory cytokines and have palliative properties in relation to inflammation or irritation, thus increasing their therapeutic potential.

The present invention provides novel compositions comprising two or three of the above-mentioned triterpenes. The compositions of the invention, or two or three of the triterpenes present in an extract obtainable from a natural source, can further be combined with any other pharmacological active agent so as to potentiate the pharmacological action.

It has been found by the present inventor that a composition comprising two or three of the triterpenes selected from the group consisting of dihydrobutyrospermol, dihydrolupeol and dihydroparkeol, or derivatives thereof, such as esters thereof, especially cinnamic acid, acetic add or fatty add esters, significantly suppresses hypersensitivity reactions when absorbed systemically following oral, parenteral or topical administration. Optionally, such composition comprises one or more pharmaceutically acceptable carrier(s) or excipient(s) suitable for allowing systemic absorption of said triterpenes.

Furthermore, it has been found by the present inventor that a pharmaceutical composition comprising two or three of the triterpenes selected from the group consisting of dihydrobutyrospermol, dihydrolupeol and dihydroparkeol and formulated for topical administration, using one or more pharmaceutically acceptable carrier(s) and/or excipient(s), significantly inhibits inflammation or hypersensitivity of the skin or mucous membranes following topical administration. Topical administration may allow the triterpenes to be present In the mucous or dermis for an adequate period to achieve the therapeutic effect.

Compared to existing therapeutic agents, such as corticosterolds or non-steroidal anti-inflammatory drugs, the pharmaceutical compositions and dietary supplements according to the present invention have the advantage of not being associated with any serious side effects, as all of their components are non-toxic and well tolerated by the organism in the pharmacologically relevant doses.

Thus, the present invention provides, In a first aspect, a composition comprising:
a mixture of triterpenes comprising two or three of the triterpenes selected from the group consisting of dihydrobutyrospermol, dihydrolupeol and dihydroparkeol, wherein said triterpenes may be in the form of the free alcoholic form such that the parenteral or in derivatised form.

In another aspect, the present invention relates to a process for the preparation of a composition comprising a mixture of triterpenes comprising two or three of the triterpenes selected from the group consisting of dihydrobutyrospermol; dihydrolupeol; and dihydroparkeol wherein said triterpenes may be in the form of the free alcoholic form or in derivatised form, comprising the steps of
i) providing an extract from a natural source comprising two or three of the triterpenes selected from the group consisting of butyrospermol, lupeol, parkeol and derivatives thereof; and
ii) hydrogenation of said extract by any suitable method of hydrogenation.

In yet another aspect, the present invention relates to a composition obtainable by the process of the invention.

The derivatives of dihydrobutyrospermol, dihydrolupeol and dihydroparkeol are such that the alcohol moiety may be derivatised. Thus, the triterpenes may be In the form of their free alcohols (OH), or in the form of ethers, esters, or as the free alkoxide ion present as a salt. As Is known to the person skilled In the art, an alcohol moiety may be easily derivatised so as to achieve a variety of pharmacological and pharmacokinetic objectives. Thus, prodrugs of the triterpenes are suitable embodiments of derivatives. Moreover, the triterpenes, either In the form of the free alcohols or In the form of derivatised triterpenes such as esters or ethers of triterpenes, may further be in the form of a pharmaceutically acceptable salt, various stereolsomeric forms, induding racemic mixtures.

Suitable embodiments of esters may be selected from the group consisting of cinnamic acid ester, acetic acid ester and fatty acid esters.

Due to the pharmacological effects relating to Immunomodulation as mentioned above, the compositions according to the invention can be employed for a number of therapeutic applications.

Accordingly, in a further aspect, the present invention relates to a composition of the invention for use as a medicament.

In a still further aspect, the present invention relates to the use of a composition of the invention for the preparation of a medicament for treating hypersensitivity and/or Inflammatory reactions in a mammal, including a human.

In an even further aspect, the present invention relates to the use of a composition of the invention for the preparation of a medicament for the treatment of hypersensitivity skin disease in a mammal, including a human.

In another aspect, the present invention relates to the use of a composition of the invention for the preparation of a medicament for the treatment of IgE mediated allergic reaction In a mammal, including a human.

In yet another aspect, the present invention relates to the use of a composition of the invention for the preparation of a medicament for the treatment of diseases selected from the group consisting of asthma, allergic rhinitis and anaphylaxis in a mammal, including a human.

In still another aspect, the present invention relates to the use of a composition of the invention for the manufacture of a medicament for the treatment of diseases selected from the group consisting of autoimmune diseases and chronic Inflammatory diseases in a mammal, including a human.

In an even further aspect, the present invention relates to the use of a composition of the invention for the manufacture of a medicament for the treatment of diseases selected from the group consisting of prostatitis and benign prostatic hypertrophy in a mammal, including a human.

In a still further aspect, the present invention relates to the use of a composition of the Invention for the manufacture of a medicament for the treatment of pain in a mammal, including a human.

In yet another aspect, the present invention relates to the use of a composition of the invention for the manufacture of a medicament for the treatment of cardiovascular diseases in a mammal, including a human.

In a further aspect, the present invention relates to the use of a composition of the invention for the manufacture of a medicament for the treatment of viral Infections in a mammal, including a human.

In a still further aspect, the present invention relates to the use of a composition of the invention for the manufacture of a medicament for the treatment of cancer in a mammal, including a human.

### DETAILED DESCRIPTION OF THE INVENTION

It has surprisingly been found that compositions containing a mixture of triterpenes comprising two or three of the triterpenes selected from the group consisting of dihydrobutyrospermol, dlhydrolupeof and dihydroparkeol, wherein said triterpenes may be in the form of free alcohols or derivatives thereof, especially esters with cinnamic add, acetic acid or fatty acids, inhibit the inflammatory cytokines, TNF-α and IL-6, and have surprising anti-inflammatory effects as well as antiviral effects. Compared to the existing antiinflammatory drugs, such as steroids and Ibuprofen, and to the antiviral drugs, the compositions of the invention have the advantage of being active against a broad range of inflammatory diseases and viruses and not being associated with any serious side effects. Furthermore, the compositions of the invention have palliative properties in relation to inflammation or irritation, thereby increasing their therapeutic potential.

The structures of dihydrobutyrospermol, dihydrolupeol and/or dihydroparkeol are shown in formula 1, II and III, respectively.

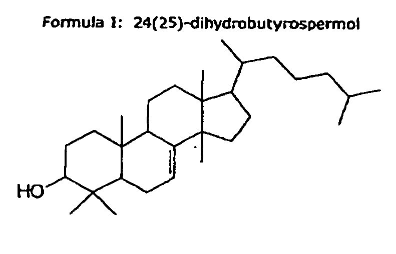

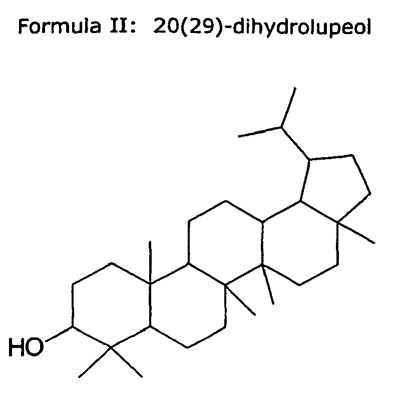

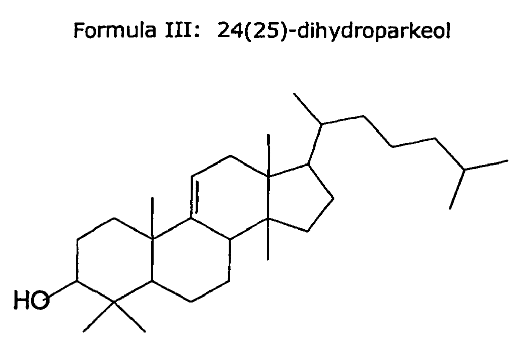

More specifically, dihydrobutyrospermol, dihydrolupeol and/or dihydroparkeol as described above provide the following pharmacological effects upon administration to the living organism:
- Suppression of inflammation, hypersensitivity and irritation
- Direct antiviral action against a broad range of pathogenic viruses
- Palliative effects on inflammation or irritation caused by the viral infection

In example 3 and 4, the topical and systemic anti-inflammatory effect of a composition according to the invention was demonstrated in a test model. The topical and systemic anti-inflammatory effect was just about that of hydrocortisone and ibuprofen, respectively. Moreover, the compositions and triterpenes according to the invention were demonstrated to inhibit the secretion of inflammatory cytokines, TNF-α and Il-6.. As is known to a person skilled in the art, given that the secretion of certain inflammatory cytokines were inhibited *in vitro* by triterpenes and compositions according to the invention, it is to be anticipated that the compositions of the invention will inhibit other cytokines which relate to anti-inflammatory and hypersensitivity effects.

The anti-viral effect of a composition according to the invention was measured in a well-established model of viral infection in cells (Herpes simplex infection in Vero cells and Influenza A Infection In MDCK cells). In this experiment the composition of the invention dose dependently Inhibited both Herpes simplex and Influenza A infection at concentrations of 8-200 µg/mL. In a separate experiment (see example 2) in rat the LD₅₀ of the same composition of the invention was shown to be above 2000 mg/kg. This indicates a very beneficial therapeutic index against viral Infections of the composition of the invention.

Thus, it has surprisingly been found by the present inventor that a composition comprising:
a mixture of triterpenes comprising two or three of the triterpenes selected from the group consisting of dihydrobutyrospermol, dihydrolupeol and dihydroparkeol, wherein said triterpenes may be in the form of free alcohols or derivatives thereof, preferably esters with cinnamic add, acetic add or fatty acids
significantly suppresses viral infections as well as inflammation or hypersensitivity reactions.

Said composition may be suitably formulated as a pharmaceutical composition for oral, topical, transdermal, or parenteral administration, preferably topical and transdermal administration. The composition may also be suitably formulated as a dietary supplement or a cosmetic.

"A" dietary supplement" is defined according to the U.S. Food and Drug Administration in the Dietary Supplement Health and Education Act of 1994 (DSHEA).

The DSHEA gives the following formal definition of a "dietary supplement": "A dietary supplement:
- is a product (other than tobacco) that is intended to supplement the diet that bears or contains one or more of the following dietary ingredients: a vitamin, a mineral, an herb or other botanical, an amino acid, a dietary substance for use by man to supplement the diet by increasing the total daily intake, or a concentrate, metabolite, constituent, extract, or combinations of these things.
- is intended for ingestion in pill, capsule, tablet, or liquid form."

Similar definitions exist In other parts of the world, e.g. in Europe. Different denominations concerning "dietary supplements" are used around the world, such as "food supplements", "neutraceuticals", "functional foods" or simply "foods". In the present context the term "dietary supplement" covers any such denomination or definition.

When applied topically the pharmaceutical composition inhibits inflammation or hypersensitivity of the skin or mucous membranes. Thus, the triterpenes is released from the composition to the dermis or mucous for being present in the dermis or mucous in sufficient amounts and/or for an adequate period of time to exhibit its pharmacological effect. For example, the composition according to the invention suppresses that kind of topical inflammation, which is also treated by the steroid, hydrocortisone 17-butyrate.

The compositions of the invention are also effective in suppression of inflammation and hypersensitivity reactions following systemic absorption. As disclosed herein, the inventors have provided evidence that the compositions according to the invention inhibit inflammation following systemic uptake.

Thus, compositions irrespective of their oral, parenteral or topical administration provide a surprisingly good anti-hypersensitivity and anti-inflammatory effect with a surprisingly good safety profile. Thus, the compositions of the invention are virtually non-toxic and yet very therapeutically effective.

The proper amount of the triterpenes in the composition in order to obtain beneficial effects may vary according to the individual needs of the patient, the severity of the symptoms and the disease. Thus, compositions according to the invention comprise dihydrobutyrospermoi, dihydrolupeol or dihydroparkeol individually or in mixtures in a weight percentage (w/w) of typically at least 0.1%, e.g. at least 0.5%, at least 1%, such as at least 2%, at least 5%, e.g. at least 10%, at least 15%, such as at least 20%, e.g. at least 25%, at least 30%, e.g. at least 35%, such as at least 40%, or at least 45%, at least 50%, e.g. at least 55%, at least 60%, or at least 65%, such as at least 70%, e.g. at least 75%, at least 80%, e.g. at least 85%, at least 90%, such as at least 91%, e.g. at least 92%, at least 93%, e.g. at least 94%, at least 95%, or at least 96%, at least 97%, e.g. at least 98%, at least 99%, e.g. at least 100%. Alternatively stated, the individual weight percentage (w/w) of dihydrobutyrospermol, dihydrolupeol or dihydroparkeol or weight percentage (w/w) of mixtures thereof In the composition typically is at most 100%, e.g. 99%, at most 98%, e.g. at most 97%, e.g. at most 96, e.g. at most 95%, e.g. at most 90%, e.g. at most 85%, e.g. at most 80%, e.g. at most 75%, e.g. at most 70%, at most 65%, e.g. at most 60%, e.g. at most 55%, e.g. at most 50%, e.g. at most 45%, at most 40%, e.g. at most 35%; e.g. at most 30%, e.g. at most 25%, e.g. at most 20%, e.g. at most 15%, e.g. at most 10%, e.g. at most 9%, e.g. at most 8%, e.g. at most 7%, e.g. at most 6%, e.g. at most 5%, e.g. at most 2%, e.g. at most 1%, e.g. at most 0.5%, e.g. at most 0.1%.

Thus, the compositions comprises a two or three of dihydrobutyrospermol, dihydrolupeol or dihydroparkeol, in an amount corresponding to weight percentage (w/w) in the range of 0.1-100%, such as in the range of 1-98%, such as in the range of 2-96%, e.g. In the range of 5-94%, such as In the range of 7-92%, such as In the range of 10-90%, e.g. In the range of 12-88%, e.g. in the range of 14-86%, such as in the range of 16-84%, such as In the range of 18-82%, e.g. in the range of 20-80%.

As stated, the compositions comprise mixtures of the triterpenes. These may be of any combination, such as comprising two or all three triterpenes. Furthermore, the mixtures may contain exclusively the triterpenes in alcoholic form or In derivatised form, or mixtures of alcoholic and derivatised form. Additionally, the mixtures may comprise several derivatives or isomeric forms of each triterpene such that the compositions of the invention may comprise two or more of dihydrobutyrospermol, dihydrolupeol and/or dihydroparkeol and/or derivatives thereof, such as 3, 4, 5, 6, 7, 8, 9 or 10 of such triterpenes, as well as mixtures comprising the triterpene alcohols as well as their derivatives. Preferred derivatives of the invention are dihydrobutyrospermyl acetate, dihydrolupeyl acetate, dihydroparkeyl acetate, dihydrobutyrospermyl cinnamate, dihydrolupeyl cinnamate, dihydroparkeyl cinnamate.

According to the invention dihydrobutyrospermol, dihydrolupeol and/or dihydroparkeol and/or the derivatives thereof may be obtained by synthetically means or being obtainable from a natural source. Thus, the said triterpenes may be produced synthetically or bio-synthetically by any suitable chemical reaction. Also according to the invention, these substances may be in the form of an extract obtainable from a natural source such as derived from plants by any suitable sequence of extraction, fractionation and optionally hydrogenation. Numerous plants may be suitable as sources for the production of dihydrobutyrospermol, dihydrolupeol and/or dihydroparkeol and/or the derivatives thereof.

Thus, in certain embodiments of the invention, the triterpene or mixture of triterpenes is an extract obtainable from a natural source selected from the group consisting of *Butyrospermum parkii, Camellia japonica, Camellia sasanqua* and *Thea sinensis.*

As known to the person skilled in the art, an extract of said triterpenes may be enriched by adding synthetically prepared triterpenes into the extract.

When deriving the compositions according to the invention from a plant, any part of the plant may be used, e.g. the fruit (nut), leaves, stem, bark or root. Especially the oils or fat of a suitable plant are relevant as sources of the triterpenes of the invention. The triterpenes of the invention may be derived from a plant by any method of extraction or fractionation, e.g. in the unsaponifiable fraction of a vegetable oil. Such extraction may be performed on fresh or dried plant material, e.g. by distillation (e.g. hydro, steam or vacuum distillation). Extraction may be performed with a number of different solvents, preferably non-polar solvents such as organic solvents. However, polar solvents such as aqueous solution or polar organic solvents may also be applicable. The extraction can be performed hot or cold by the employment of any extraction technology, e.g. maceration, percolation or supercritical extraction (e.g. with carbon dioxide).

Non-limiting examples of preferred extraction solvents are acetone, methyl ethyl ketone, methyl acetate, ethyl acetate, lower alkanols having 1-4 carbon atoms, pentane, hexane, heptane and mixtures thereof. The preferred extraction temperature is close to the boiling point of the employed solvent due to extraction efficacy, but lower temperatures are also applicable, a longer period of extraction then being necessary.

By changing the composition of the applied solvent, the extraction can be made more selective for certain constituents thus enhancing or reducing the contents thereof in the finished extract or concentrate.

After the primary extraction process, a second step of processing, such as liquid-liquid extraction, precipitation, column chromatography or any type of distillation, can be employed to remove or to concentrate any constituent of the extract. Hereby any constituent can be avoided or concentrated in the finished composition according to the invention. Thus the content of any component can be standardised and the ratio between dihydrobutyrospermol, dihydrolupeol and/or dihydroparkeol and/or the derivatives thereof may be varied dramatically in the compositions of the invention, and in specific cases any of the compounds may be excluded from a specific composition according to the invention.

In a preferred embodiment of the invention, the triterpenes of the invention are extracted as butyrospermol, lupeol and/or parkeol and/or the derivatives thereof and subsequently hydrogenated by any suitable method of hydrogenation into dihydrobutyrospermol, dihydrolupeol and/or dihydroparkeol and/or the derivatives thereof, the said triterpenes being obtained from the following vegetable sources but not limiting to: *Butyrospermum parkii, Camellia japonica, Camellia sasanqua* or *Thea sinensis.*

When the triterpenes being derived from plants, the compositions or extracts of the invention may contain other triterpenes than dihydrobutyrospermol, dihydrolupeol and/or dihydroparkeol and/or the derivatives thereof. Non-limiting examples of such triterpenes are butyrospermol, lupeol, parkeol, germanicol, dammaradienol, 24-methylene-dammarenol, α-amyrin, β-amyrin and faradiol. Also, the compositions of the invention may contain sterols, of which non-limiting examples are β-sitosterol, campesterol, brassicasterol, stigmasterol, avanasterol, 24-methyl-cholest-7-enol, karitesterol A, karitesterol B and α-spinasterol. Such additional triterpene alcohols and sterols may be in any form, e.g. in the form of free alcohols or derivatives thereof, especially cinnamic acid esters, acetic acid esters or fatty acid esters.

In pharmaceutical compositions, dietary supplements or cosmetics according to the invention, such additional triterpenes and sterols may quantitatively exceed the content of dihydrobutyrospermol, dihydrolupeol and/or dihydroparkeol and/or the derivatives thereof. In some cases such additional triterpenes and sterols may contribute significantly to the pharmacological effects of the compositions of the invention. Thus according to the invention, additive or synergistic effects may occur between dihydrobutyrospermol, dihydrolupeol and/or dihydroparkeol and the optional additional triterpenes and sterols.

The pharmacological actions as described *supra* provide part of the rationale for the following therapeutic applications of the triterpenes of the invention or of a composition according to the present invention.

Thus, in a second aspect the invention relates to the use of a triterpene or mixture of triterpenes selected from the group consisting of dihydrobutyrospermol, dihydrolupeol and dihydroparkeol, or derivatives thereof for the preparation of a medicament, a cosmetic or a dietary supplement for immunomodulation of a mammal, such as a human. Preferably, the medicament comprises a composition as defined herein.

In a still further aspect, the triterpenes or the composition according to the invention may be administered to an individual for treating relevant diseases according to the invention.

Thus, the invention relates to a method for immunomodulation in a mammal, such as a human, comprising the administration to said mammal a triterpene or mixture of triterpenes selected from the group consisting of dihydrobutyrospermol, dihydrolupeol and dihydroparkeol, or derivatives thereof.

The immunomodulation according to the invention is selected from the group consisting of suppression of hypersensitivity and suppression of inflammatory reactions. Typically, the immunomodulation is associated with diseases and disorders selected from the group consisting of viral infections, bacterial infection, hypersensitivity skin diseases, atopic eczema, contact dermatitis, seborrhoeic eczema, psoriasis, IgE mediated allergic reactions, asthma, allergic rhinitis, anaphylaxis, autoimmune disease, chronic inflammatory disease, Crohn's disease, ulcerative colitis, proctitis, rheumatoid arthritis, gout, asteoarthritis, prostatitis, benign prostatic hyperthrophy, cardiovasculary diseases, hyperlipidemia or aetherosclerosis, pain and cancer.

The said triterpene or mixture of triterpenes comprised in said medicament may be selected from the group consisting of dihydrobutyrospermol, dihydrolupeol and dihydroparkeol or derivatives thereof are selected from the free alcohol and an ester derivative of the triterpenes selected from the group consisting of dihydrobutyrospermol, dihydrolupeol and dihydroparkeol. Furthermore, said said triterpene or mixture of triterpenes comprised in said medicament may be obtained by synthetically means or be an extract obtainable from a natural source selected from the group consisting of *Butyrospermum parkii, Camellia japonica, Camellia sasanqua* and *Thea sinensis*.

Thus, embodiments according to the invention such as i) compositions comprising said triterpenes or, II) use of said triterpenes for preparation of a medicament for immunomodulation In a mammal, relate to the following diseases, disorders or conditions that involves immunomodulation:
- Hypersensitivity and/or inflammatory reactions. According to the Invention an known conditions and diseases associated with inflammation and hypersensitivity reactions are relevant including I-Iv type hypersensitivity and those caused by direct histamine release, and the following examples are not limiting with respect to this: infections (viral, bacterial, fungal, parasitic, etc.), cold and flu, contact dermatitis, insect bites, allergic vasculitis, postoperative reactions, transplantation rejection (graft-versus-host disease), asthma, eczema (e.g. atopic dermatitis), urticaria, allergic rhinitis, anaphylaxis, autoimmune hepatitis, Primary biliary cirrhosis, Primary sclerosing cholangitis, Autoimmune hemolytic anemias, Grave's disease, Myasthenia gravis, Type 1 Diabetes Mellitus, Inflammatory myopathies, Multiple sclerosis, Hashimoto's thyreolditis, Autoimmune adrenalitis, Crohn's Disease, Ulcerative Colitis, Glomerulonephritis, Progressive Systemic Sclerosis (Scieroderma), Sjögren's Disease, Lupus Erythematosus, Primary vasculitis, Rheumatoid Arthritis, Juvenile Arthritis, Mixed Connective Tissue Disease, Psorlasis, Pemfigus, Pemfigold, Dermatitis Herpetiformis, etc.
- Inflammation and/or hypersensitivity of the skin, such as dermis and mucous. This effect can be obtained in relation to any skin disease or In relation to any disease that causes such symptoms of the skin. Examples of such conditions are but not limited to atopic eczema, contact dermatitis, seborrhoeic eczema, infections and/or psoriasis.
- IgE mediated allergic reactions and conditions. The applicant puts forward the hypothesis that the therapeutic action is due to the suppressing effect on hypersensitivity reaction of the above mentioned compositions. The therapeutic action may be relevant to all known IgE mediated allergic reactions and conditions, and the following examples are not limiting with respect to this: asthma, eczema (e.g. atopic dermatitis), urticaria, allergic rhinitis, anaphylaxis, etc.
- Autoimmune diseases and/or chronic Inflammatory diseases. The applicant puts forward the hypothesis that the therapeutic action is due to the immuno-modulating and suppressing effect on hypersensitivity reactions of the above mentioned composition. The therapeutic action may be relevant to all known autoimmune disorders, and the following examples are not limiting with respect to this:
   Autoimmune hepatitis, Primary biliary cirrhosis, Primary sclerosing cholangitis, Autoimmune hemolytic anemias, Grave's disease, Myasthenia gravis, Type 1 Diabetes Mellitus, Inflammatory myopathies, Multiple sclerosis, Hashimoto's thyreoiditis, Autoimmune adrenalitis, Crohn's Disease, Ulcerative Colitis, Glomerulonephritis, Progressive Systemic Sclerosis (Scleroderma), Sjögren's Disease, Lupus Erythematosus, Primary vasculitis, Rheumatoid Arthritis, Juvenile Arthritis, Mixed Connective Tissue Disease, Psoriasis, Pemfigus, Pemfigoid, Dermatitis Herpetiformis, etc.
- Viral or bacterial infections. Specifically, the viral infections may be caused by the vira as described herein. The viral infections relate to a broad spectrum of viral infections, in particular herpes infections, especially herpes simplex infections such as herpes labialis, common cold or influenza, viral pharyngitis, viral pneumonia, viral hepatitis, bronchitis or other diseases associated with inflammation or irritation in the respiratory system. Bacterial infections may also be treated with these triterpenes, since said infections are often secondary to viral infections and viral infections may also be secondary infections as the result of bacterial infections. Thus, a bacterial Infection may often only be effectively treated by treating the viral infection.
- Prostatitis or benign prostatic hypertrophy, inflammation of various tissues, e.g. inflammation of the prostate, in particular prostatitis. "Prostatitis" is defined as inflammatory conditions affecting the prostate, including acute and chronic infections with specific bacteria and, more commonly, instances in which signs and symptoms of prostatic inflammation are present but no specific organism can be detected. Accordingly, the compositions of the invention may also be employed for the management of benign prostatic hypertrophy, a condition associated with swelling of the prostate.
- Cardiovascular disease, especially hyperlipidemia and atherosclerosis.
- Cancer
- Alleviation of pain. The applicant puts forward the hypothesis that the therapeutic action is related to immunomodulation, possibly to a suppressing effect on hypersensitivity reactions.

Besides these specific therapeutic areas, the action of the above mentioned triterpenes and compositions is relevant to all known conditions and diseases associated with hypersensitivity reaction, and the following examples are not limiting with respect to this: infections (viral, bacterial, fungal, parasitic, etc.), cold and flu, contact dermatitis, insect bites, allergic vasculitis, postoperative reactions, transplantation rejection (graft-versus-host disease), etc.

Without being limited to a specific theory, the suppression of Inflammatory and hypersensitivity reactions by the triterpenes and composition according to the invention may, at least In part, relate to the inhibition of the secretion of inflammatory cytokines. Specifically, the present inventors provide evidence for the inhibition of seaetlon of TNF-α and IL-6. However, as is known to the skilled person, other inflammatory cytokines also exist. Thus, given that the secretion of those other cytokines Is inhibited by the triterpenes and compositions according to the invention, it is anticipated that inflammatory diseases and hypersensitivity reactions related to those cytokines may be suppressed by the present compositions and triterpenes.

In interesting embodiments according to the invention, the composition of the invention may be used for the preparation of a medicament for treatment of viral infections such as those caused by various types of herpes simplex or other viruses as discussed herein. Moreover, in some cases the viral infections may be related to influenza or just some symptoms of influenza.

However, the potential viral targets are many and highly different but a distinction of viruses in RNA viruses and DNA viruses is obvious. Important families of DNA viruses are the herpes viruses such as Herpes simplex virus (HSV). Two types of HSV have been identified: HSV-1 is the cause of herpes labialis (cold sore), herpetic stomatitis, keratoconjunctivitis and encephalitis, whereas H5V-2 causes genital herpes and may also be responsible for systemic infection. This partitioning of HSV-1 versus HSV-2 viruses are not rigld because HSV-1 can cause genital herpes and HSV-2 can give rise to pharyngitis. Approximately 70% of the population is infected with HSV-1 and recurrent infections occur in one third of patients.

Another member of the herpes virus family is Varicella zoster virus (VZV). VZV causes two distinct diseases: varicella (chickenpox) and herpes zoster (shingles).

Yet another member of the herpes virus family is cytomegalovirus (CMV). Infection with CMV is found world-wide and has its most profound effect as an opportunistic infection In immunocompromised individuals, particularly in recipients of bone marrow or solid organ transplants and in patients with AIDS. Ninety percent of patients with AIDS are infected with CMV.

Another member of the herpes virus family is Epstein-Barr virus (EBV). This virus causes an acute febrile illness known as mononucleosis (glandular fever), which occurs world-wide in adolescents and young adults. The symptoms are fever, headache, malaise and sore throat. The disease is commonly associated with mild hepatitis.

Yet another member of the herpes virus family is human herpes virus type 6 (HHV-6). This virus, which occurs world-wide, infects T-lymphocytes and exists as a latent infection in over 90% of the adult population. HHV-6 causes roseola infantum (exanthem subitum) which manifests itself as a high fever followed by generalised macular rash in infants. In immunocompromised individuals, the virus may lead to severe pneumonia.

Another family of DNA viruses are the adenoviruses. Adenovirus infection commonly presents itself as an acute pharyngitis. In adults adenovirus may cause acute follicular conjunctivitis and more rarely pneumonia.

Yet another family of DNA viruses are the papovaviruses. These small viruses tend to produce chronic infections. An important member of the papovaviruses is human papillomavirus (HPV). There are many types of HPV, which are responsible for the common wart and have been implicated in the aetiology of carcinoma of the cervix (types 16 and 18). Other members of the papovavirus family are BK virus (a polyomavirus) and JC virus which occur in immunosuppressed individuals.

Another family of DNA viruses are the parvoviruses. An important member of this family is human parvovirus B19 which produces erythema infectiosum (fifth disease), a common infection in school children. A chronic infection with anaemia occurs in pcompromised subjects.

An important family of RNA viruses are the picornaviruses. Among these the polioviruses cause poliomyelitis which occurs when a susceptible person is infected with poliovirus types 1, 2 or 3. Other members of the picornavirus family are coxsackievirus, echovirus and enterovirus, which each have a number of different subtypes, and are all spread by the faecal-oral route. They are responsible for a broad spectrum of diseases involving the skin, mucous membranes, muscles, nerves, the heart and rarely other organs, such as the liver and pancreas. An especially important member of the picornavirus family is rhinovirus among which there are more than 120 subtypes. Rhinoviruses are responsible for common colds, and due to the many immunotypes, vaccine control is not practically possible.

Another family of RNA viruses are the reoviruses. Reovirus infection occurs mainly in children and causes mild respiratory symptoms and diarrhoea. A member of the reovirus family, the rotavirus, is responsible world-wide for both sporadic cases and epidemics of diarrhoea and is presently one of the most important causes of childhood diarrhoea.

Yet another family of RNA viruses are the togaviruses. Among these rubella virus is the cause of German measles. While the disease occurs sporadically world-wide, epidemics are not uncommon. Another member of the togavirus family is arbovirus. Over 385 viruses are classified as arboviruses. They are zoonotic viruses, with the possible exception of O'nyong-nyong virus of which humans are the only known vertebrate hosts. Although most arbovirus diseases are generally mild, epidemics are frequent and when they occur the mortality is high. A group of arboviruses are the alphaviruses which are all transmitted by mosquitoes. Human infection is characterised by fever, skinrash, myalgia and sometimes encephalitis. Another group of arboviruses are the flaviviruses, some of which are transmitted by ticks and others by mosquitoes. Yellow fever is one of the best known flavivirus diseases and is of widely varying severity. It is characterised by high fever and the mortality rate is up to 40% in severe cases. Another flavivirus disease is dengue which is found mainly in Africa and Asia. The disease is usually endemic, but epidemics have been reported.

Japanese encephalitis is a mosquito-borne encephalitis caused by flavivirus. It has been reported most frequently in the rice-growing countries of South East Asia and the Far East. As with other viral infections the clinical manifestations are variable. Mortality varies from 7 to 40% and is higher in children.

Another group of RNA viruses are the bunyaviruses which form a family of more than 200 viruses, most of which are arthropod-borne. Congo-Crimean haemorrhagic fever, which is caused by bunyavirus is found mainly in Asia and Africa. The symptoms are influenza-like, with fever and haemorrhagic manifestations. The mortality is 10-50%. A specific type of bunyavirus is hantavirus. The hantaviruses are enzootic and spread by aerosolized excretions. The most severe disease caused by hantaviruses is Korean haemorrhagic fever with a mortality of 5-10%. In the United States, a new hantavirus causes the acute respiratory distress syndrome (ARDS).

Another important group of RNA viruses are the orthomyxoviruses of which there are three important types: influenza A, B and C. Influenza A is responsible for pandemics and epidemics. Influenza B often causes smaller or localised and milder outbreaks, e.g. In schools. Influenza C rarely produces disease In humans.

Yet another group of RNA viruses are the paramyxoviruses. Among these parainfluenza virus types I to IV have a world-wide distribution causing the disease parainfluenza with features that are quite similar to common cold. Another paramyxovirus is the cause of measles (rubeola) which is a highly communicable disease that occurs world-wide. Yet another paramyxovirus is the cause of mumps which is spread by droplet infection, by direct contact. The symptoms are non-specific and include fever, malaise, headache and anorexia. Respiratory syncytial virus is a paramyxovirus that causes many respiratory infections in epidemics each winter. It is a common cause of bronchiolitis In Infants, which is complicated by pneumonia in approximately 10% of cases.

Another group of RNA viruses are the rhabdoviruses. Among these the rabies virus is a major problem in some countries and carries a high mortality.

Yet another group of RNA viruses are the retrovtruses which are distinguished from other RNA viruses by their ability to replicate through a DNA intermediate using an enzyme, reverse transcriptase. Two retroviruses, HIV-1 and HIV-2 (the cause of AIDS), are classified as lentiviruses because of their slow disease progress. Another retrovirus is HTLV-1 which causes tropical spastic paraparesis.

Another group of RNA viruses are the arenaviruses. The prototype virus of this group is lymphocytic choriomeningitis. This infection is a zoonosis, the natural reservoir being the house mouse. The disease is characterised by fever, myalgia and headache and can In some cases give encephalitis. Another disease caused by arenaviruses is lassa fever. The disease is characterised by non-specific symptoms and fever. Death occurs in 15-20% of hospitalised patients.

Marburg virus disease and Ebola virus disease which are both RNA viruses are mentioned here together because they both give rise to febrile illnesses with similar clinical manifestations. The illness is characterised by the acute onset of strong headache, severe myalgia and high fever followed by prostration. Diarrhoea is profuse and is associated with abdominal cramps and vorniting. Mortality is high.

Interesting embodiments according to the invention relates to those, wherein the composition or medicament further comprises one or more therapeutically active agents, or where the triterpenes are administered along with one or more therapeutically active agents so as to potentiate the therapeutic action.

As stated, the triterpenes may be sultably formulated for oral, parenteral, transdermal, transmucosal or topical administration. The pharmaceutical compositions for oral, topical, transdermal, transmucosal or parenteral administration may be In form of, e.g., solid, semi-solid or fluid compositions and formulated according to conventional pharmaceutical practice, see, e.g., "Remington: The science and practice of pharmacy" 20^{th} ed. Mack Publishing, Easton PA, 2000 ISBN 0-912734-04-3 and "Encyclopedia of Pharmaceutical Technology", edited by Swarbrick, J. & J. C. Boylan, Marcel Dekker, Inc., New York, 1988 ISBN 0-8247-2800-9.

According to the invention, the triterpenes may be absorbed systemically following oral, parenteral or topical administration of said composition. In an interesting embodiment, the triterpenes exhibit their therapeutic action locally in the dermis or mucous following topical administration of said composition or triterpenes.

The choice of pharmaceutically acceptable exdplents or carriers for use according to the invention and the optimum concentration thereof is determined on the basis of the selection of the triterpenes, the kind of dosage form chosen and the mode of administration. However, a person skilled In the art of pharmaceutical formulation may find guidance In e.g., "Remington: The science and practice of pharmacy" 20^{th} ed. Mack Publishing, Easton PA, 2000 ISBN 0-912734-04-3. A pharmaceutically acceptable excipient is a substance, which is substantially harmless to the individual to which the composition will be administered. Such an excipient suitably fulfils the requirements given by the national drug agencies. Official pharmacopeias such as the British Pharmacopeia, the United States of America Pharmacopeia and the European Pharmacopeia set standards for well-known pharmaceutically acceptable excipients.

For topical, trans-mucosal and trans-dermal compositions, such as administration to the mucosa or the skin (dermis), the compositions for use according to the Invention may contain conventional non-toxic pharmaceutically acceptable carriers and excipients including microspheres and liposomes.

Topical administration includes generally any administration to a dermis or a mucosa, such as mucosa in the lung, nose, mouth, gastrointestinal tract, vagina, uterus and rectum. Trans-dermal and trans-mucosal administration are variations of topical administration forms, where the triterpenes penetrate the dermis or mucosa, either by passive means (such as diffusion) or by actively transporting the triterpenes through the dermis or mucosa.

The topical, trans-mucosal and trans-dermal compositions for use according to the invention include an array of solid, semi-solid and fluid compositions. Compositions of particular relevance are e.g. pastes, ointments, hydrophilic ointments, creams, gels, hydrogels, solutions, emulsions, suspensions, lotions, liniments, resoriblets, suppositories, enema, pessaries, moulded pessaries, vaginal capsules, vaginal tablets, shampoos, jellies, soaps, sticks, sprays, powders, films, foams, pads, sponges (e.g. collagen sponges), pads, dressings (such as, e.g., absorbent wound dressings), drenches, bandages, plasters and transdermal delivery systems.

Oral compositions according to the invention include an array of solid, semi-solid and fluid compositions. Compositions of particular relevance are e.g. solutions, suspensions, emulsions, uncoated tablets, immediate-release tablets, modified-release tablets, gastro-resistant tablets, orodispersible tablets, efferverscent tablets, chewable tablets, soft capsules, hard capsules, modified-release capsules, gastro-resistant capsules, uncoated granules, effervescent granules, granules for the preparation of liquids for oral use, coated granules, gastro-resistant granules, modified-release granules, powders for oral adminstration and powders for the preparation of liquids for oral use.

Administration by the parenteral route includes intravenous, intraarticular, intraventricular, intracapsular, intraspinal, intramuscular, subcutaneous, intradermal, buccal, sublingual, nasal, rectal, vaginal or transdermal routes. Compositions of particular relevance are e.g. liquids, emulsions, suspensions, oils, efferverscent tablets, chewable tablets and implants.

Pharmaceutically acceptable carriers and/or excipients can be water or vehicles other than water, and said other vehicles can be used in the compositions and can include solids or liquids such as solvents, thickeners and powders. Examples of each of these types of vehicles, which can be used singly or as compositions of one or more vehicles, are as follows:
Emollients, such as stearyl alcohol, glyceryl monoricinoleate, glyceryl monostearate, propane-1,2-diol, butane-1,3-diol, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, cetyl palmitate, dimethylpolysiloxane, di-n-butyl sebacate, isopropyl myristate, isopropyl palmitate, isopropyl stearate, butyl stearate, polyethylene glycol, triethylene glycol, lanolin, castor oil, acetylated lanolin alcohols, petroleum, mineral oil, butyl myristate, isostearic acid, palmitic acid, isopropyl linoleate, lauryl lactate, myristyl lactate, decyl oleate, myristyl myristate;
typically solvents, such as water, methylene chloride, isopropanol, castor oil, ethylene glycol monoethyl ether, diethylene glycol monobutyl ether, diethylene glycol monoethyl ether, dimethyl sulfoxide, tetrahydrofuran, vegetable and animal oils, glycerol, ethanol, propanol, propylene glycol, and other glycols or alcohols, fixed oils;
humectants or moistening agents, such as glycerin, sorbitol, sodium 2-pyrrolidone-5-carboxylate, soluble collagen, dibutyl phthalate, gelatin;
powders, such as chalk, talc, kaolin, starch and derivatives thereof, gums, colloidal silicon dioxide, sodium polyacrylate, chemically modified magnesium aluminium silicate, hydrated aluminium silicate, carboxyvinyl polymer, sodium carboxymethyl cellulose, ethylene glycol monostearate;
gelling or swelling agents, such as pectin, gelatin and derivatives thereof, cellulose derivatives such as methyl cellulose, carboxymethyl cellulose or oxidised cellulose, cellulose gum, guar gum, acacia gum, karaya gum, tragacanth gum, bentonite, agar, alginates, carbomer, gelatine, bladderwrack, ceratonia, dextran and derivatives thereof, ghatti gum, hectorite, ispaghula husk, xanthan gum;
polymers, such as polylactic acid or polyglycolic acid polymers or copolymers thereof, paraffin, polyethylene, polyethylene oxide, polyethylene glycol, polypropylene glycol, polyvinylpyrrolidone;
surfactants, such as non-ionic surfactants, e.g. glycol and glycerol esters, macrogol ethers and esters, sugar ethers and esters, such as sorbitan esters, ionic surfactants, such as amine soaps, metallic soaps, sulfated fatty alcohols, alkyl ether sulfates, sulfated oils, and ampholytic surfactants and lecithins;
buffering agents, such as sodium, potassium, aluminium, magnesium or calcium salts (such as the chloride, carbonate, bicarbonate, citrate, gluconate, lactate, acetate, gluceptate or tartrate).

Furthermore, it is obvious that in the use according to the invention for the preparation of medicaments or dietary supplements, the above mentioned compositions may be mixed with additives such as surfactants, solvents, thickeners, stabilisers, preservatives, antioxidants, flavours, etc. to obtain a desirable product formulation suitable for systemic or topical administration. Similarly, a pharmaceutical or dietary supplement according to the invention may further contain such additives. Optionally, the composition may also contain surfactants such as bile salts, polyoxyethylene-sorbitan-fatty acid esters or polyalcohol mixed chain-length fatty acid esters for improving dispersibility of the composition in the digestive fluids leading to improved bioavailability or for obtaining the final dosage form of the composition.

In addition to the formulations described *supra,* the compositions of the invention may also be formulated such that the release rate of the triterpenes and/or the optionally one or more active agent(s) is controlled, such as for quick release, sustained release, delayed release, slow release. Thus, the carrier or composition may be formulated as a depot preparation. Such long acting formulations may be administered by implantation (for example subcutaneously or intramuscularly) or by intramuscular injection. Thus, for example, the compositions may be formulated with suitable polymeric or hydrophobic materials (for example as an emulsion in an acceptable oil) or ion exchange resins, or as sparingly soluble derivatives, for example, as a sparingly soluble salt.

Alternatively, other pharmaceutical delivery systems may be employed. Liposomes and emulsions are well known examples of delivery vehicles that may be used to deliver compositions of the invention. Additionally, the compositions may be delivered using a sustained-release system, such as semi-permeable matrices of solid polymers containing the therapeutic agent. Various sustained-release materials have been established and are well known by those skilled in the art. Sustained-release capsules may, depending on their chemical nature, release the triterpenes from the composition for a few hours, through days and weeks up to over 100 days.

### EXAMPLES

### Example 1

### Summary of the study

A composition according to the invention containing 3.1% (w/w) dihydrobutyrospermyl acetate and 2.8% (w/w) dihydrolupeyl acetate formulated in triglyceride was evaluated for possible antiviral effects against Herpes simplex virus and Influenza A virus in Monkey kidney (Vero) cells and MDCK cells, respectively (plaque assay).

The composition according to the invention inhibited both viruses dose-dependently with an IC₅₀ of 40-200 µg/mL.

### Test substance

A composition according to the invention was prepared by hydrogenation and fractionation of shea butter *(Butyrospermum parkii)*. The composition was analysed by GC-MS, EI in full scan mode. A HP-5 column 30m, ID 0.25mm, 0.25µm film thickness (5% diphenyl, 95% dimethylpolysiloxane) was used. The sample was dissolved in ethyl acetate (1 mg/mL) and the components were quantitated using stigmasterol as internal standard (0.02 mg/mL solution in ethyl acetate). The composition was found to contain 3.1% (w/w) of dihydrobutyrospermyl acetate and 2.8% (w/w) dihydrolupeyl acetate.

A stock solution of the composition of the invention was prepared for the viral experiments containing 10 mg/mL in ethanol. The compound required sonication and mild warming to dissolve. The solution remained opalescent. Upon cooling, the compound precipitated. This precipitate was easily redissolved.

### Test system

1) Vero Cells for Herpes simplex type 1 virus.
2) MDCK cells for Influenza type A virus.

### Cytotoxicity test

The stock solution was diluted in Hank's balanced salt solution (HBSS) to concentrations of 1000 µg/mL and 200 µg/mL. The ethanol solvent was also diluted to equivalent concentrations (i. e. 10% and 2%). Duplicate wells of 6-well plates of monkey kidney (Vero) cells and MDCK cells were inoculated with diluted compound and diluted solvent. The inoculum was removed and 3mL of medium added. The cells were examined daily for three days for cytotoxic effects.

### Methods

The stock solution and solvent were each diluted in HBSS. The concentrations covered were 2000, 400, 80, 16, 3.2 and 0.64 µg/mL compound and the corresponding 20, 4, 0.8, 0.16, 0.032 and 0.0064% ethanol. Equal volumes of diluted stock virus (Herpes and Influenza) were mixed with an equal volume of diluted compound or ethanol. Herpes and Influenza were mixed with dilutions covering the range 400 to 0.64 µg/mL (4 to 0.0064% ethanol).

Triplicate wells of 6-well plates of Vero or MDCK cells were inoculated (with the Herpes or. Influenza samples, respectively), with the compound (or ethanol) virus mixtures. Three wells were inoculated with HBSS spiked with the input virus dilution. Cells inoculated with HBSS served as the negative control. After adsorption, the inocula were removed, cells rinsed once with 3mL of HBSS, and 3mL of agarose overlay medium added back. Upon development of plaques in the positive control wells, the wells were fixed and stained with Giemsa.

### Results

The composition Inhibited both Herpes virus and Influenza virus dose-dependently with an IC-50 between 40 and 200 µg/mL in both cases. No cytotoxicity was observed at the doses employed.

### Example 2

### Summary

The composition according to the invention described in example 1 was evaluated for acute oral toxicity in the rat. At a dose of 2000 mg/kg, the substance was found not to produce toxicity or mortality. Thus it was concluded that the LD₅₀ was above 2000 mg/kg body weight.

### Test substance

The composition according to the invention described in example 1 was used in this experiment.

### Study description

The acute oral toxicity in rats was determined according to the method recommended in the OECD guideline No 420, "Acute Oral Toxicity - Fixed Dose Method", July 1992 and the EEC Directive published in: "Official Journal of the European Communities" No: L 383A, volume 35, 29.12.1992, part B1 "Acute Toxicity (Oral) - Fixed Dose Method".

The study was initiated with a sighting study, in which one female rat was given 2000 mg composition/kg body weight. No clinical signs of toxicity were observed in this rat.

On the basis of the results of the sighting study the main study was carried out with one group consisting of 5 female rats given a dose of 2000 mg composition/kg body weight.

All animals in the main study survived the treatment and showed no signs of evident toxicity. The rats had a normal body weight gain during the study period.

Under the experimental conditions described in this report, it was found that the dose level tested (2000 mg composition/kg body weight), the highest dose level required, did not produce mortality. The minimal lethal dose was above 2000 mg composition/kg body weight.

### Example 3

### Summary

The composition according to the invention described in example 1 was evaluated for topical antiinflammatory effect in the mouse phorbolester ear oedema test.
The composition according to the invention inhibited ear oedema significantly at both doses tested.

### Objective

After having demonstrated in a separate experiment that the composition according to the invention described in example 1 *in vitro* inhibits the secretion of inflammatory cytokines (TNF-α and IL-6) in lipolysaccharide-stimulated peritoneal macrophages (mouse), it was decided to test the efficacy of the composition *in vivo*, topically administered in the tetradecanoyl phorbol acetate (TPA) induced ear Inflammation test in the mouse, a commonly employed method for screening and evaluation of antiinflammatory drugs. Locoid® cutaneous solution (0.1% hydrocortisone 17-butyrate) is used as a positive control.

### Test articles and vehicle

The test articles are the composition according to the invention described in example 1 (Compound 1) and Locoid® cutaneous solution (hydrocortisone 17-butyrate) obtained from a pharmacy in Denmark. The test article is dissolved in acetone, which is used as vehicle.

### Animals

The study is performed in female SPF NMRI mice of the stock Bom:NMRI from M & B A/S, DK-8680 Ry.

### Housing

The study will take place in an animal room provided with filtered air. The temperature in the room is set at 21 - 23°C and the relative humidity to ≥50%. The room is Illuminated to give a cycle of 12 hours light and 12 hours darkness. Light is on from 06.00 till 18.00 h. The animals is housed in Macrolon type III cages (40x25x14 cm), nine in each cage. The cages is cleaned and the bedding changed at least once a week. The animal room is cleaned and disinfected with Diversol Bx.

### Bedding

The bedding is sawdust (Tapvei 4HV) from Tapvei Oy, 73620 Kortteinen, Finland.

### Diet

A complete pelleted rodent diet "Altromin 1314" from Chr. Petersen, DK- 4100 Ringsted, is available *ad libitum.*

### Drinking water

The animals will have free access to bottles with domestic quality drinking water added citric acid to pH 3.

### Animal randomisation and allocation

On the day of arrival the animals is randomly allocated to groups, each of 10 mice.

### Animal and cage identification

Each animal is identified by coloured marks on the tails. Each cage is marked with study number 2021, cage number, group number and animal numbers.

### Body weight

The animals are weighed on day -1.

### Procedure

The test substances are applied in 20 µl volumes to the inner surface of the right ear on day 0. 20 minutes before and again 20 minutes after TPA treatment. All groups are treated with 20 µl acetone on the left ear and with 20 µl TPA, 400 µg/ml, on the right ear.

The groups and doses are as follows:

| Group | Drug, left/right ear | Dose, mg per application |
|---|---|---|
| A | -/Vehicle | - |
| B | Compound 1 | 1.0 |
| C | Compound 1 | 5.0 |
| D | Hydrocortisone 17-but. | 0.02 |

Three hours after the TPA application the mice are sacrificed, the ears cut off and weighed. Mean weights and standard deviations are calculated. Percent inhibition of the oedema compared with group A is calculated for the B, C and D.

### Findings

Ear swelling is determined as the difference between the weight of right and left ear. Compound 1 gave an inhibition of ear swelling of 76% and 52% at 5.0 mg/ear and 1.0 mg/ear, respectively (p<0.05, Wilcoxon). Hydrocortisone 17-butyrate solution gave an inhibition of ear swelling of 90% (p<0.05, Wilcoxon).

### Conclusion

Compound 1 inhibited ear swelling dose-dependently and in the higher dose of the same size of order as the effect seen after hydrocortisone 17-butyrate. This finding is noteworthy because Compound 1 is not associated with the unpleasant adverse effects associated with glucocorticoids such as hydrocortisone 17-butyrate.

### Example 4

### Summary

The composition according to the invention described in example 1 was evaluated for systemic antiinflammatory effect in the carrageenin-induced paw oedema test in the rat. The composition according to the invention had a significant and dose-dependent antiinflammatory effect.

### Objective

After having demonstrated in a separate experiment that a composition according to the invention (Compound 2) *in vitro* inhibits the secretion of inflammatory cytokines (TNF-α and IL-6) in lipolysaccharide-stimulated peritoneal macrophages (mouse), it was decided to test the efficacy of the substance *in vivo,* systemically administered In the carrageenin-induced paw oedema test in the rat, a commonly employed method for screening and evaluation of antiinflammatory drugs. Carrageenin, the phlogistic agent of choice for testing antiinflammatory drugs, is a mucopolysaccharide derived from Irish sea moss, *Chondrus.* Ibuprofen is used as a positive control.

### Test article and vehicle

The test article is the composition according to the invention described in example 1 further modified so that the dihydro-triterpenes are predominantly in the form of fatty acid esters (Compound 2). Ibuprofen is obtained from Astion A/S, Denmark.
The test article is dissolved in peanut oil, which is used as vehicle.

### Animals

The study is performed in male SPF Sprague Dawley rats of the stock Mol:SPRD from M & B A/S, Tornbjergvej 40, DK-4623 Lille Skensved. At start of the acclimatisation period the rats are in the weight range of 80 - 100 g.

### Housing

The study takes place in an animal room provided with filtered air. The temperature in the room is set at 21 -23°C and the relative humidity to ≥ 50%. The room is illuminated to give a cycle of 12 hours light and 12 hours darkness. Light is on from 06.00 till 18.00 h.

The animals are housed in Macrolon type III cages (40x25x14 cm) six in each cage. The cages will be cleaned and the bedding changed at least once a week. The animal room is cleaned and disinfected with Diversol Bx.

### Bedding

The bedding is sawdust (Tapvei 4HV) from Tapvei Oy, 73620 Kortteinen, Finland.

### Diet

A complete pelleted rodent diet "Altromin 1314" from Chr. Petersen, DK- 4100 Ringsted, is available *ad libitum*.

### Drinking water

The animals will have free access to bottles with domestic quality drinking water added citric acid to pH 3.

### Animal randomisation and allocation

On the day of arrival the animals will be randomly allocated to groups, each of 10 rats.

### Animal and cage identification

Punched earmarks identify each animal. Each cage is marked with study number 2022, cage number, group number and animal numbers.

### Body weight

The animals are weighed on days: -2 and 0 of dosing.

### Dosing

The test articles or vehicle are administered orally by gavage in volumes of 20 ml per kg body weight, 0-5 minutes before injection of carrageenin into the foot.
The groups and dose levels are as follows:

| | | |
|---|---|---|
| Group | Test article | Dose, mg/kg |
| A | Vehicle - control | - |
| B | Compound 2 | 1000 |
| C | Compound 2 | 500 |
| D | Compound 2 | 250 |
| E | Ibuprofen | 150 |

Carrageenin (from Sigma) is prepared as a 1% suspension in sterile 0.9% NaCl -solution. A volume of 0.1 ml is injected through a 25-gauge needle into the plantar tissue of the right hind paw of the rats within 5 minutes after treatment with the test articles.

### Measurements

Immediately before the dosing and carrageenin injection and three and five hours later the foot volume is measured using a plethysmometer LE 7500 from Letica Scientific Instruments, Spain.

### Clinical signs

All visible signs of ill health and any behavioural changes is recorded daily during the study. Any deviation from normal is recorded with respect to time of onset, duration and intensity.

### Findings

After three hours an inhibition of 63, 59 and 44% of the paw oedema was seen after 1000, 500, and 250 mg/kg Compound 2, respectively (p<0.05, Wilcoxon). After five hours an inhibition of 75, 56 and 44% of the paw oedema was seen after 1000, 500, and 250 mg/kg Compound 2, respectively (p<0.05, Wilcoxon). At 150 mg/kg ibuprofen gave an inhibition of paw oedema of 81 and 88% after three and five hours respectively (p<0.05, Wilcoxon).

### Interpretation

Compound 2 inhibited paw oedema significantly and dose-dependently, and in the higher dose of the same size of order as the effect seen after 150 mg/kg Ibuprofen. This finding is noteworthy because Compound 2 is not associated with the unpleasant adverse effects associated with non-steroidal antiinflammatory drugs such as ibuprofen.

## Claims

1. A composition comprising:
A mixture of triterpenes comprising two or three of the triterpenes selected from the group consisting of dihydrobutyrospermol; dihydrolupeol; and dihydroparkeol, wherein said triterpenes may be in the form of the free alcoholic form or in derivatised form.

2. The composition according to claim 1, wherein said derivative Is an ester.

3. The composition according to claim 2, wherein said ester is selected from the group consisting of cinnamic acid esters, acetic acid esters and fatty acid esters.

4. The composition according to any one of preceding claims, wherein the mixtures of triterpenes is in an amount of at least of 1.0% by weight.

5. The composition according to claim 4, wherein the mixture of triterpenes is In an amount of at least 2%.

6. The composition according to claim 5, wherein the mixture of triterpenes Is in an amount of at least 5%.

7. The composition according to claim 6, wherein the mixture of triterpenes is in an amount of at least 10%.

8. The composition according to any one of the preceding claims, wherein said mixture of triterpenes Is in an extract obtainable from a natural source.

9. The composition according to claim 8, wherein the natural source is selected from the group consisting of *Butyrospermum parkii, Camellia japonica, Camellia sasanqua* and *Thea sinensis.*

10. The composition according to any of the preceding claims, wherein said composition comprises one or more acceptable excipient(s) and/or carrier(s).

11. The composition according to any of the preceding claims, wherein said composition is formulated as a pharmaceutical composition.

12. The composition according to claim 11 formulated for oral, topical, transdermal, or parenteral administration.

13. The composition according to claim 12, formulated for oral administration.

14. The composition according to claim 13, wherein said composition is formulated as a soft or hard capsule.

15. The composition according to claim 12, formulated for topical administration.

16. The composition according to any of claims 1-10, wherein said composition is formulated as a dietary supplement.

17. The composition according to any of claims 1-10, wherein said composition is formulated as a cosmetic.

18. A process for the preparation of a composition comprising a mixture of triterpenes comprising two or three of the triterpenes selected from the group consisting of dihydrobutyrospermol; dihydrolupeol; and dihydroparkeol wherein said triterpenes may be in the form of the free alcoholic form or in derivatised form, comprising the steps of
i) providing an extract from a natural source comprising two or three of the triterpenes selected from the group consisting of butyrospermol, lupeol, parkeol and derivatives thereof; and
II) hydrogenation of said extract by any suitable method of hydrogenation.

19. The process according to claim 18, wherein said hydrogenation Is such that the composition comprises said mixture of triterpenes in a weight percentage (w/w) of at least 1.0%.

20. The process according to claim 19, wherein said hydrogenation is such that the composition comprises said mixture of triterpenes in a weight percentage (w/w) of at least 2.0%.

21. The process according to claim 20, wherein said hydrogenation is such that the composition comprises said mixture of triterpenes in a weight percentage (w/w) of at least 5.0%.

22. The process according to claim 21, wherein said hydrogenation is such that the composition comprises said mixture of triterpenes In a weight percentage (w/w) of at least 10.0%.

23. The process according to any of claims 18-22, wherein the natural source Is selected from the group consisting of *Butyrospermum parkii, Camellia japonica, Camellia sasanqua* and *Thea sinensis*.

24. A composition obtainable by the process defined in any of claims 18-23.

25. A composition as defined in any of claims 1-17 or 24 for use as a medicament.

26. Use of a composition as defined in any of claims 1-17 or 24 for the preparation of a medicament for treating hypersensitivity and/or inflammatory reactions In a mammal, including a human.

27. Use of a composition as defined in any of claims 1-17 or 24 for the preparation of a medicament for the treatment of hypersensitivity skin disease in a mammal, including a human.

28. The use according to claim 27, wherein said hypersensitivity skin disease Is selected from the group consisting of atopic eczema, contact dermatitis, seborrhoeic eczema and psoriasis.

29. Use of a composition as defined in any of claims 1-17 or 24 for the preparation of a medicament for the treatment of IgE mediated allergic reaction in a mammal, including a human.

30. Use of a composition as defined in any of claims 1-17 or 24 for the preparation of a medicament for the treatment of diseases selected from the group consisting of asthma, allergic rhinitis and anaphylaxis in a mammal, including a human.

31. Use of a composition as defined in any of claims 1-17 or 24 for the preparation of a medicament for the treatment of diseases selected from the group consisting of autoimmune diseases and chronic inflammatory diseases in a mammal, Including a human.

32. The use according to claim 31, wherein the autoimmune disease or chronic inflammatory disease is selected from the group consisting of diabetes, Crohn's disease, ulcerative colitis, rheumatoid arthritis, gout and osteoarthritis.

33. The use according to claim 32, wherein the autoimmune diseases or chronic inflammatory disease is selected from the group consisting of rheumatoid arthritis, gout and osteoarthritis.

34. Use of a composition as defined in any of claims 1-17 or 24 for the preparation of a medicament for the treatment of diseases selected from the group consisting of prostatitis and benign prostatic hypertrophy in a mammal, including a human.

35. Use of a composition as defined in any of claims 1-17 or 24 for the preparation of a medicament for the treatment of pain in a mammal, including a human.

36. Use of a composition as defined in any of claims 1-17 or 24 for the preparation of a medicament for the treatment of cardiovascular diseases in a mammal, including a human.

37. The use according to claim 36, wherein the cardiovascular disease is selected from the group consisting of hyperlipidemia and atherosclerosis.

38. Use of a composition as defined in any of claims 1-17 or 24 for the preparation of a medicament for the treatment of viral infections in a mammal, including a human.

39. The use according to claim 38, wherein the viral Infection is influenza and symptoms of influenza.

40. The use according to claim 38, wherein the viral infection is herpes.

41. Use of a composition as defined in any of claims 1-17 or 24 for the preparation of a medicament for the treatment of cancer in a mammal, including a human.

## Patentansprüche

1. Zusammensetzung, umfassend:
Mischung von Triterpenen, welche Mischung zwei oder drei der Triterpene umfasst, die aus der Gruppe bestehend aus Dihydrobutyrospermol;
Dihydrolupeol; und Dihydroparkeol ausgewählt sind, worin die Triterpene in der Form der freien alkoholischen Form oder in derivatisierter Form sein können.

2. Die Zusammensetzung nach Anspruch 1, worin das Derivat ein Ester ist.

3. Die Zusammensetzung nach Anspruch 2, worin der Ester aus der Gruppe bestehend aus Zimtsäureestern, Essigsäureestern und Fettsäurestern ausgewählt ist.

4. Die Zusammensetzung nach irgendeinem der vorhergehenden Ansprüche, worin die Mischungen von Triterpenen in einer Menge von mindestens 1,0 Gewichtsprozent sind.

5. Die Zusammensetzung nach Anspruch 4, worin die Mischung von Triterpenen in einer Menge von mindestens 2% ist.

6. Die Zusammensetzung nach Anspruch 5, worin die Mischung von Triterpenen in einer Menge von mindestens 5% ist.

7. Die Zusammensetzung nach Anspruch 6, worin die Mischung von Triterpenen in einer Menge von mindestens 10% ist.

8. Die Zusammensetzung nach irgendeinem der vorhergehenden Ansprüche, worin die Mischung von Triterpenen in einem Extrakt ist, der aus einer natürlichen Quelle erhältlich ist.

9. Die Zusammensetzung nach Anspruch 8, worin die natürliche Quelle aus der Gruppe bestehend aus *Butyrospermum parkii, Camellia japonica, Camellia sasanqua* und *Thea sinensis* ausgewählt ist.

10. Die Zusammensetzung nach irgendeinem der vorhergenden Ansprüche, worin die Zusammensetzung ein oder mehrere akzeptable Bindemittel und/oder Trägermittel umfasst.

11. Die Zusammensetzung nach irgendeinem der vorhergehenden Ansprüche, worin die Zusammensetzung als eine pharmazeutische Zusammensetzung formuliert ist.

12. Die Zusammensetzung nach Anspruch 11, welche Zusammensetzung für orale, topische, transdermale oder parenterale Verabreichung formuliert ist.

13. Die Zusammensetzung nach Anspruch 12, für orale Verabreichung formuliert.

14. Die Zusammensetzung nach Anspruch 13, worin die Zusammensetzung als eine weiche oder harte Kapsel formuliert ist.

15. Die Zusammensetzung nach Anspruch 12, für topische Verabreichung formuliert.

16. Die Zusammensetzung nach irgendeinem der Ansprüche 1-10, worin die Zusammensetzung als ein Nahrungsergänzungsmittel formuliert ist.

17. Die Zusammensetzung nach irgendeinem der Ansprüche 1-10, worin die Zusammensetzung als ein Kosmetikum formuliert ist.

18. Verfahren zur Herstellung einer Zusammensetzung, umfassend eine Mischung von Triterpenen, welche Mischung zwei oder drei der Triterpene umfasst, die aus der Gruppe bestehend aus Dihydrobutyrospermol; Dihydrolupeol; und Dihydroparkeol ausgewählt sind, worin die Triterpene in der Form der freien alkoholischen Form oder in derivatisierter Form sein können, umfassend folgende Stufen:
i) Bereitstellung eines Extrakts aus einer natürlichen Quelle, welcher Extrakt zwei oder drei der Triterpene umfasst, die aus der Gruppe bestehend aus Butyrospermol, Lupeol, Parkeol und Derivaten davon ausgewählt sind; und
ii) Hydrierung des Extrakts durch irgendein geeignetes Hydrierungsverfahren.

19. Das Verfahren nach Anspruch 18, worin die Hydrierung so ist, dass die Zusammensetzung die Mischung von Triterpenen in einem Gewichtsprozent (Gew.-%) von mindestens 1,0% umfasst.

20. Das Verfahren nach Anspruch 19, worin die Hydrierung so ist, dass die Zusammensetzung die Mischung von Triterpenen in einem Gewichtsprozent (Gew.-%) von mindestens 2,0% umfasst.

21. Das Verfahren nach Anspruch 20, worin die Hydrierung so ist, dass die Zusammensetzung die Mischung von Triterpenen in einem Gewichtsprozent (Gew.-%) von mindestens 5,0% umfasst.

22. Das Verfahren nach Anspruch 21, worin die Hydrierung so ist, dass die Zusammensetzung die Mischung von Triterpenen in einem Gewichtsprozent (Gew.-%) von mindestens 10,0% umfasst.

23. Das Verfahren nach irgendeinem der Ansprüche 18-22, worin die natürliche Quelle aus der Gruppe bestehend aus *Butyrospermum parkii, Camellia japonica, Camellia sasanqua* und *Thea sinensis* ausgewählt ist.

24. Zusammensetzung, die durch das Verfahren, das in irgendeinem der Ansprüche 18-23 definiert ist, erhältlich ist.

25. Zusammensetzung, wie in irgendeinem der Ansprüche 1-17 oder 24 definiert, zur Anwendung als ein Arzneimittel.

26. Anwendung einer Zusammensetzung, wie in irgendeinem der Ansprüche 1-17 oder 24 definiert, zur Herstellung eines Arzneimittels zur Behandlung von Hypersensitivität und/oder inflammatorischen Reaktionen in einem Säugetier, einschließlich eines Menschen.

27. Anwendung einer Zusammensetzung, wie in irgendeinem der Ansprüche 1-17 oder 24 definiert, zur Herstellung eines Arzneimittels zur Behandlung von überempfindlicher Hautkrankheit in einem Säugetier, einschließlich eines Menschen.

28. Die Anwendung nach Anspruch 27, wo die überempfindliche Hautkrankheit aus der Gruppe bestehend aus atopischem Ekzem, Kontaktdermatitis, seborroischem Ekzem und Psoriasis ausgewählt ist.

29. Anwendung einer Zusammensetzung, wie in irgendeinem der Ansprüche 1-17 oder 24 definiert, zur Herstellung eines Arzneimittels zur Behandlung von IgEvermittelnder allergischer Reaktion in einem Säugetier, einschließlich eines Menschen.

30. Anwendung einer Zusammensetzung, wie in irgendeinem der Ansprüche 1-17 oder 24 definiert, zur Herstellung eines Arzneimittels zur Behandlung von Krankheiten, die aus der Gruppe bestehend aus Asthma, Rhinitis allergica und Anaphylaxie in einem Säugetier, einschließlich eines Menschen, ausgewählt sind.

31. Anwendung einer Zusammensetzung, wie in irgendeinem der Ansprüche 1-17 oder 24 definiert, zur Herstellung eines Arzneimittels zur Behandlung von Krankheiten, die aus der Gruppe bestehend aus autoimmunen Krankheiten und chronischen inflammatorischen Krankheiten in einem Säugetier, einschließlich eines Menschen, ausgewählt sind.

32. Die Anwendung nach Anspruch 31, worin die autoimmune Krankheit oder chronische inflammatorische Krankheit aus der Gruppe bestehend aus Diabetes, Crohns Krankheit, ulcerative Colitis, rheumatoid Arthritis, Gicht und Osteoarthritis ausgewählt ist.

33. Die Anwendung nach Anspruch 32, worin die autoimmunen Krankheiten oder chronische inflammatorische Krankheit aus der Gruppe bestehend aus rheumatoid Arthritis, Gicht und Osteoarthritis ausgewählt sind.

34. Anwendung einer Zusammensetzung, wie in irgendeinem der Ansprüche 1-17 oder 24 definiert, zur Herstellung eines Arzneimittels zur Behandlung von Krankheiten, die aus der Gruppe bestehend aus Prostatitis und gutartiger prostatischer Hypertrophie in einem Säugetier, einschließlich eines Menschen, ausgewählt sind.

35. Anwendung einer Zusammensetzung, wie in irgendeinem der Ansprüche 1-17 oder 24 definiert, zur Herstellung eines Arzneimittels zur Behandlung von Schmerzen in einem Säugetier, einschließlich eines Menschen.

36. Anwendung einer Zusammensetzung, wie in irgendeinem der Ansprüche 1-17 oder 24 definiert, zur Herstellung eines Arzneimittels zur Behandlung von kardiovaskulären Krankheiten in einem Säugetier, einschließlich eines Menschen.

37. Die Anwendung nach Anspruch 36, worin die kardiovaskuläre Krankheit aus der Gruppe bestehend aus Hyperlipidämie und Atherosklerose.

38. Anwendung einer Zusammensetzung, wie in irgendeinem der Ansprüche 1-17 oder 24 definiert, zur Herstellung eines Arzneimittels zur Behandlung von viralen Infektionen in einem Säugetier, einschließlich eines Menschen.

39. Die Anwendung nach Anspruch 38, worin die virale Infektion Influenza und Symptome von Influenza ist.

40. Die Anwendung nach Anspruch 38, worin die virale Infektion Herpes ist.

41. Anwendung einer Zusammensetzung, wie in irgendeinem der Ansprüche 1-17 oder 24 definiert, zur Herstellung eines Arzneimittels zur Behandlung von Krebs in einem Säugetier, einschließlich eines Menschen.

## Revendications

1. Une composition comprenant :
Un mélange de triterpènes comprenant deux ou trois des triterpènes choisis dans le groupe consistué par le dihydrobutyrospermol; le dihydrolupeol; et le dihydroparkeol, où lesdits triterpènes peuvent être sous la forme de la forme alcool libre ou sous forme dérivée.

2. La composition selon la revendication 1, où ledit dérivé est un ester.

3. La composition selon la revendication 2, où ledit ester est choisi dans le groupe consistué par les esters d'acide cinnamique, les esters d'acide acétique et les esters d'acides gras.

4. La composition selon une quelconque des revendications précédentes, où les mélanges de triterpènes sont présents en quantité d'au moins 1,0% en masse.

5. La composition selon la revendication 4, où le mélange de triterpènes est présent en quantité d'au moins 2%.

6. La composition selon la revendication 5, où le mélange de triterpènes est présent en quantité d'au moins 5%.

7. La composition selon la revendication 6, où le mélange de triterpènes est présent en quantité d'au moins 10%.

8. La composition selon une quelconque des revendications précédentes, où ledit mélange de triterpènes est dans un extrait obtensible à partir d'une source naturelle.

9. La composition selon la revendication 8, où la source naturelle est choisie dans le groupe consistué par *Butyrospermum parkii, Camellia japonica, Camellia sasanqua* et *Thea sinensis.*

10. La composition selon une quelconque des revendications précédentes, où ladite composition comprend un ou plusieurs excipient(s) et/ou porteur(s) acceptable(s).

11. La composition selon une quelconque des revendications précédentes, où ladite composition est formulée en tant que composition pharmaceutique.

12. La composition selon la revendication 11 formulée pour administration orale, topicale, transdermale ou parentérale.

13. La composition selon la revendication 12, formulée pour administration orale.

14. La composition selon la revendication 13, où ladite composition est formulée en tant que capsule molle ou dure.

15. La composition selon la revendication 12, formulée pour administration topicale.

16. La composition selon une quelconque des revendications 1-10, où ladite composition est formulée en tant que supplément diététique.

17. La composition selon une quelconque des revendications 1-10, où ladite composition est formulée en tant que cosmétique.

18. Un procédé pour la préparation d'une composition comprenant un mélange de triterpènes comprenant deux ou trois des triterpènes choisis dans le groupe consistué par le dihydrobutyrospermol; le dihydrolupeol; et le dihydroparkeol, où lesdits triterpènes peuvent être sous la forme de la forme alcool libre ou sous forme dérivée, comprenant les étapes de
i) production d'un extrait à partir d'une source naturelle comprenant deux ou trois des triterpènes choisis dans le groupe consistué par le butyrospermol, le lupeol, le parkeol et les dérivés de ceux-ci ; et
ii) hydrogénation dudit extrait par n'importe quelle méthode d'hydrogénation appropriée.

19. Le procédé selon la revendication 18, où ladite hydrogénation est telle que la composition comprend ledit mélange de triterpènes dans un pourcentage massique (m/m) d'au moins 1,0%.

20. Le procédé selon la revendication 19, où ladite hydrogénation est telle que la composition comprend ledit mélange de triterpènes dans un pourcentage massique (m/m) d'au moins 2,0%.

21. Le procédé selon la revendication 20, où ladite hydrogénation est telle que la composition comprend ledit mélange de triterpènes dans un pourcentage massique (m/m) d'au moins 5,0%.

22. Le procédé selon la revendication 21, où ladite hydrogénation est telle que la composition comprend ledit mélange de triterpènes dans un pourcentage massique (m/m) d'au moins 10,0%.

23. Le procédé selon une quelconque des revendications 18-22, où la source naturelle est choisie dans le groupe consistué par *Butyrospermum parkii, Camellia japonica, Camellia sasanqua* et *Thea sinensis.*

24. Une composition obtensible par le procédé défini dans une quelconque des revendications 18-23.

25. Une composition telle que définie dans une quelconque des revendications 1-17 ou 24 pour usage en tant que médicament.

26. L'usage d'une composition telle que définie dans une quelconque des revendications 1-17 ou 24 pour la préparation d'un médicament pour le traitement de l'hypersensibilité et/ou les réactions inflammatoires chez un mammifère, y compris un humain.

27. L'usage d'une composition telle que définie dans une quelconque des revendications 1-17 ou 24 pour la préparation d'un médicament pour le traitement des dermatopathies d'hypersensibilité chez un mammifère, y compris un humain.

28. L'usage selon la revendication 27, où ladite dermatopathie d'hypersensibilité est choisie dans le groupe consistué par l'eczéma atopique, la dermatite de contact, l'eczéma séborrhéique et le psoriasis.

29. L'usage d'une composition telle que définie dans une quelconque des revendications 1-17 ou 24 pour la préparation d'un médicament pour le traitement de la réaction allergique médiée par l'IgE chez un mammifère, y compris un humain.

30. L'usage d'une composition telle que définie dans une quelconque des revendications 1-17 ou 24 pour la préparation d'un médicament pour le traitement de maladies choisies dans le groupe consistué par l'asthme, la rhinite allergique et les anaphylaxies chez un mammifère, y compris un humain.

31. L'usage d'une composition telle que définie dans une quelconque des revendications 1-17 ou 24 pour la préparation d'un médicament pour le traitement de maladies choisies dans le groupe consistué par les maladies autoimmunes et les maladies inflammatoires chroniques chez un mammifère, y compris un humain.

32. L'usage selon la revendication 31, où la maladie autoimmune ou la maladie inflammatoire chronique est choisie dans le groupe consistué par le diabète, la maladie de Crohn, la colite ulcérative, l'arthrite rhumatoïde, la goutte et l'ostéoarthrite.

33. L'usage selon la revendication 32, où la maladie autoimmune ou la maladie inflammatoire chronique est choisie dans le groupe consistué par l'arthrite rhumatoïde, la goutte et l'ostéoarthrite.

34. L'usage d'une composition telle que définie dans une quelconque des revendications 1-17 ou 24 pour la préparation d'un médicament pour le traitement de maladies choisies dans le groupe consistué par la prostatite et l'hypertrophie prostatique bénigne chez un mammifère, y compris un humain.

35. L'usage d'une composition telle que définie dans une quelconque des revendications 1-17 ou 24 pour la préparation d'un médicament pour le traitement de la douleur chez un mammifère, y compris un humain.

36. L'usage d'une composition telle que définie dans une quelconque des revendications 1-17 ou 24 pour la préparation d'un médicament pour le traitement de maladies cardiovasculaires chez un mammifère, y compris un humain.

37. L'usage selon la revendication 36, où la maladie cardiovasculaire est choisie dans le groupe consistué par l'hyperlipidémie et l'athérosclérose.

38. L'usage d'une composition telle que définie dans une quelconque des revendications 1-17 ou 24 pour la préparation d'un médicament pour le traitement d'infections virales chez un mammifère, y compris un humain.

39. L'usage selon la revendication 38, où l'infection virale est la grippe et les symptômes de la grippe.

40. L'usage selon la revendication 38, où l'infection virale est l'herpès.

41. L'usage d'une composition telle que définie dans une quelconque des revendications 1-17 ou 24 pour la préparation d'un médicament pour le traitement du cancer chez un mammifère, y compris un humain.
